# EUROPEAN PATENT APPLICATION

(11) **EP 1 813 269 A1**
(43) Date of publication of application: **01.08.2007**
(21) Application number: 05805135.0
(22) Date of filing: 21.10.2005
(51) Int. Cl.: A61K 31/122, A61K 36/00, A61P 1/04, A61P 1/16, A61P 1/18, A61P 3/10, A61P 9/00, A61P 9/10, A61P 9/12, A61P 11/00, A61P 11/06, A61P 17/00, A61P 19/02, A61P 21/04, A61P 25/00, A61P 25/16, A61P 25/28, A61P 27/02, A61P 27/12, A61P 29/00

(54) **TRANSCRIPTIONAL FACTOR Nrf2 ACTIVATOR AND FOOD HAVING THE FUNCTION OF THE SAME IMPARTED THERETO**

(30) Priority: 22.10.2004 JP 2004307838
(71) Applicant: KIRIN BEER KABUSHIKI KAISHA, Tokyo 104-8288 (JP)
(72) Inventor: SHIMURA, Masako c/o Central Lab. for Frontier Tech, Kanazawa-ku, Yokohama-shi 2360004 (JP); YOSHIDA, Aruto c/o Central Lab. for Frontier Techn, Kanazawa-ku, Yokohama-shi 2360004 (JP)
(74) Representative: HOFFMANN EITLE
(86) International application number: PCT/JP2005/019415
(87) International publication number: WO 2006/043671

(57) **Abstract**

A composition, an Nrf2 activating agent, and a food according to the present invention comprise isohumulones or isomerized hop extract as an active ingredient. They are useful in treating, preventing, or ameliorating a disease of condition which is treatable, preventable, or ameliorable by the activation of transcription factor Nrf2. More specifically, the composition, the Nrf2 activating agent and the food according to the present invention are useful in treating, preventing, ameliorating, or alleviating chronic diseases which are believed to be caused or exacerbated by cellular damages due to oxidative stress in the body or environmental substances (for example, arteriosclerosis, hypertension, diabetes, cerebral nerve degenerative diseases, skin diseases, eye diseases, asthma, and cancer) or delaying progress of these diseases, or in detoxificating xenobiotic substances.

## Description

### CROSS-REFERENCE TO RELATED APPLICATIONS

This application is based upon and claims the benefit of priority from the prior Japanese Patent Application No. 2004-307838 (filed on October 22, 2004), the entire contents of which are incorporated herein by reference.

### BACKGROUND OF THE INVENTION

### Field of the Invention

The present invention relates to a pharmaceutical agent capable of activating the transcription factor Nrf2 (NF-E2 related factor 2) and a food having such function imparted thereto. The present invention also relates to a composition and a food for use in treating, preventing, or ameliorating a disease or condition which can be treated, prevented, or ameliorated by activating the transcription factor Nrf2.

### Related Art

Oxygen is essential for humans but reactive oxygen species generated in the body as its by-product is highly toxic. It is known that cellular damages due to oxidative stress caused by reactive oxygen species are deeply involved in onset or progress of various age-related chronic diseases (for example, arteriosclerosis, hypertension, diabetes, cerebral nerve degenerative diseases, skin diseases, eye diseases, asthma, and cancer). Enhancing the capability in protecting from oxidative stress is useful in preventing these diseases or delaying their progress (Biomed Pharmacother. 57, 251-60 (2003)).

Recently, it has been revealed that a series of proteins called phase II enzymes collaborately undertake protection of the body from oxidative stress or detoxification of xenobiotic substances. Examples of known phase II enzymes include glutathione S-transferase (GST), NAD(P)H:quinone oxidoreductase 1 (NQO1), glutamyl-cysteinyl ligase (GCL), heme oxygenase 1 (HO1), and thioredoxin reductase 1 (TXNRD1). A common sequence called antioxidant responsive element (ARE) is present in a promoter of each gene of these phase II enzymes, and it is about to be revealed that its expression is induced by the transcription factor Nrf2 (NF-E2 related factor 2) (Annu Rev Pharmacol Toxicol. 43:233-60 (2003)). It is generally believed that Nrf2 is present in the cytoplasm as a Keapl-Nrf2 complex and when an activator acts therein, it moves into the nucleus and forms a heterodimer with a small Maf protein to bind ARE, thereby augmenting the expression of phase II enzymes.

As an Nrf2 activator, sulforaphane contained in cruciferous vegetables, such as broccoli, is well known. It has been reported that sulforaphane can prevent or ameliorate hypertension (Proc Natl Acad Sci USA. 101, 7094-9 (2004)), inflammations (Proc Natl Acad Sci USA. 101, 7094-9 (2004)), age-related macular degeneration (Proc Natl Acad Sci USA. 101, 10446-51 (2004)), and carcinogenesis (Proc Natl Acad Sci USA. 89, 2399-403 (1994); Carcinogenesis. 21, 2287-91 (2000)). Examples of other known Nrf2 activators include avicins (triterpenoid saponins isolated from Acacia victoriae in the pulse family) (Proc Natl Acad Sci USA. 98, 11551-6 (2001); J Clin Invest. 113, 65-73 (2004)) and 15-deoxy-D^{12,14}-prostaglandin J₂ (15dPGJ₂) (Mol Cell Biol. 24, 36-45 (2004)). These Nrf2 activators are expected to be effective in preventing or ameliorating various diseases.

The decrease in the capability in protecting the body from oxidative stress caused, for example, by the decrease in the amount of glutathione increases the level of reactive oxygen species such as superoxide in the blood and occasionally causes hypertension. This is because superoxide reacts with NO (nitrogen monoxide), which has vascular smooth muscle relaxing activity, to produce peroxynitrite, thereby preventing the relaxing activity. Further, peroxynitrite is a strong inflammation-causing substance and a possible link of its production to arteriosclerotic lesion formation has also been suggested. For example, it has been reported that in spontaneously hypertensive rats (SHRs), the level of glutathione, which plays a central role in eliminating reactive oxygen species in the vascular smooth muscle, is low and thus oxidative stress is increased (J Hypertens. 19, 1819-25 (2001)). However, since the amount of glutathione can be increased by applying sulforaphane onto vascular smooth muscle of the SHRs, amelioration of hypertension can be expected. Recently, it has been confirmed that in stroke-prone SHRs (SHRSPs), a blood pressure increase can be suppressed and vascular endothelial functions can be improved by administering broccoli sprouts containing a large amount of glucoraphanin (a metabolic precursor of sulforaphane) (Proc Natl Acad Sci USA. 101, 7094-9 (2004)). These findings show that the increase in the amount of glutathione and the induction of phase II enzymes are effective in ameliorating hypertension.

Since the brain has a relatively high metabolic rate and a low cell regenerative capacity, it is known as an organ vulnerable to damages due to oxidative stress as compared to other organs. Further, since the amount of reactive oxygen species in the brain increases with age, it is suggested that the capability in protecting the brain from oxidative stress may decrease with age. The increase in oxidative stress is believed to be closely related to the onset of cerebral nerve diseases such as Alzheimer's disease, Parkinson's disease, and amyotrophic lateral sclerosis. For example, accumulation of 4-hydroxynonenal or malondialdehyde, which is a marker for lipid peroxides, has been observed in the cerebral cortex or hippocampus of Alzheimer's disease patients, the substantia negra of Parkinson's disease patients, and the spinal fluid of amyotrophic lateral sclerosis patients. Further, it is known that in Alzheimer's disease, β-amyloid, which is believed to be a causative substance, increases the amount of intracellular hydrogen peroxide and that in Parkinson's disease, increasing dopamine is known to increase the amount of multiple reactive oxygen species molecular species including hydrogen peroxide. Further in Parkinson's disease, it has been reported that the amount of glutathione in the substantia negra makes a biochemical index for the disease and the amount of glutathione decreases with the aggravation of the disease (Nat Med. 10, Suppl S18-25 (2004)). Accordingly, augmentation of the capability in the protection from reactive oxygen species, for example, in preventing cellular lesions due to hydrogen peroxide and in increasing the amount of intracellular glutathione is considered to be effective in preventing or ameliorating these cerebral nerve degenerative diseases. In fact, it has also been reported that dimethyl fumarate and tert-butylhydroquinone which induce phase II enzymes suppress cell death due to dopamine and hydrogen peroxide (J Biol Chem. 277, 388-94 (2002); J Neurochem. 71, 69-77 (1998); Neurosci Lett. 273, 109-12 (1999)). Further, it has been revealed that for such suppression, augmentation of the expression of a part of phase II enzymes such as quinone reductase (QR) and GST is insufficient and augmentation of the expression of a series of phase II enzymes by Nrf2 activation is necessary.

Known examples of the eye diseases associated with oxidative stress include age-related macular degeneration, cataracts, light retinopathy, and retinopathy of prematurity. Age-related macular degeneration is a typical eye disease which occurs with aging to cause blindness in an aged population. Photooxidative stress on the retinal pigment epithelium has been suggested as a cause of this disease and the accumulation of oxidized products of lipids and proteins is known to be a risk factor. When photooxidative stress by a combination of retinaldehyde and long-wavelength UVA radiation is given to the retinal pigment epithelium, the amount of peroxidized lipids in the cells increases to cause cell death; however, sulforaphane capable of inducing phase II enzymes is known to suppress it. Similarly, bis(2-hydroxybenzylidene)acetone capable of inducing phase II enzymes is also shown to suppress cell death and it has thus been revealed that the capability of inducing phase II enzymes such as NQO1 and GCL and the capability of protecting from oxidative stress are important for its prevention (Proc Natl Acad Sci USA. 101, 10446-51 (2004)). Further, curcumin capable of inducing phase II enzymes is known to reduce oxidative stress and suppress galactose-induced cataracts (Mol Vis. 9, 223-30 (2003)) and further thioredoxin whose expression is increased by Nrf2 activation can be used for amelioration of light retinopathy (Invest Ophthalmol Vis Sci. 43, 1162-7 (2002)).

Cancer (malignant tumor) is today one of the major causes of death. For many years, intake of green and yellow vegetables has been said to reduce the risk of carcinogenesis. Examples of typical green and yellow vegetables include the cruciferous vegetables such as broccoli, kale, and red cabbage; it is known that when they are given to mice or rats, QR activity and GST activity in the tissue are increased. Sulforaphane is identified in broccoli as a QR activity inducer (Proc Natl Acad Sci USA. 89, 2399-403 (1992)) and is confirmed to show a strong carcinogenesis suppressing effect in the 9,10-dimethyl-1,2-benzanthracene (DMBA)-induced rat breast cancer model (Proc Natl Acad Sci USA. 89, 2399-403 (1994)) and in the azoxymethane (AOM)-induced rat colon cancer model (Carcinogenesis. 21, 2287-91 (2000)). Presumably, when these carcinogenesis-suppressing effects are exhibited, carcinogenic substances are effectively detoxificated or oxidative stress is suppressed by the increase in the amounts of phase II enzymes and glutathione induced by sulforaphane. Therefore, substances having similar activities are considered to be useful for cancer prevention. On the other hand, it has been reported that application of avicins derived from plants of the legume family onto the skin has a suppressive effect in mouse skin cancer induced with DMBA/TPA (phorbol 12-tetradecanoate 13-acetate) by hydrogen peroxide production suppression and anti-inflammatory action (Natl Acad Sci USA. 98, 11551-6 (2001)). Further, avicins have been revealed to activate Nrf2 and increase the expression of NQO1 and HO1 and the amount of glutathione and their application on the skin has been reported to suppress skin disorders such as hyperplasia or DNA damages due to ultraviolet beam (UVB) exposure (J Clin Invest. 113, 65-73 (2004)).

A pollutant, DEP (diesel exhaust particles), is known to generate oxidative stress in respiratory organs and cause symptoms such as asthma. In a mouse model in which DEP and OVA (ovalbumin) are inhaled, intraperitoneal administration of bucillamine or N-acetylcysteine has been shown to markedly suppress oxidative stress such as lipid peroxidation generated in the lung (J Immunol. 168, 2560-7 (2002)). Further, it has also been reported that Nrf2-deficient mice suffer strongly from oxidative stress due to DEP in the lung and DNA damages increase (Toxicol Appl Pharmacol. 173, 154-60 (2001)) and also that HO1, NQO1, and GST induced by Nrf2 activation are responsible for the protection from oxidative stress due to DEP and toxicity of quinone in DEP (J Immunol. 173, 3467-81 (2004)). From these findings, use of Nrf2 activators in preventing or ameliorating asthma caused by environmental pollutants and the like has been believed to be promising. Further, as for gene polymorphism for phase II enzymes whose expression is induced by Nrf2, it has been reported that the risk for bronchial hypersensitivity or asthma markedly increases in humans lacking GSTP1 activity (Am J Respir Crit Care Med. 161, 1437-42 (2000)) and that the risk for asthma increases in children lacking glutathione S-transferase mu1 (GSTM1) and NQO1 activity (Am J Respir Crit Care Med. 168, 1199-204 (2003)). Thus, the importance of protection by Nrf2 activation in asthma has been reported.

Augmentation of intracellular antioxidative activity and induction of phase II enzymes such as HO-1 by Nrf2 activation are also known to act anti-inflammatorily. For example, in administration of broccoli sprouts containing a large amount of glucoraphanin in the abovementioned SHRSPs, suppression of the infiltration of activated macrophages in the vascular wall of the aorta and the carotid artery, the ventricle, and the renal medulla are also observed, which suggest that the increase in the amount of glutathione and the induction of phase II enzymes exhibit the anti-inflammatory action. Further, adhesion and infiltration of such monocytic cells to the vascular wall are phenomena which also occur in the early stage of arteriosclerosis and are led to macrophage foam cell formation, and thus the prevention of arteriosclerosis by Nrf2 activation has been expected (Proc Natl Acad Sci USA. 101, 7094-9 (2004)). In arteriosclerosis, an increase in blood cholesterol and inflammation generated in the blood vessel are said to be the two major factors (Nat Med. 8, 1211-7 (2002)) and so far development of LDL-cholesterol lowering agents such as statins has primarily been preceded. In future, use of foods and drugs having anti-inflammatory activity will draw attention. However, there are yet many unknown points in terms of the mechanism of the suppression of monocytic cell adhesion by Nrf2 activation. It has been reported that in an in vitro experiment using vascular endothelial cells, the expression of the adhesion molecule VCAM-1 by stimulation of the inflammatory cytokine TNF-α is suppressed by augmentation of Nrf2 or NQO1 expression and action of an antioxidative substance, N-acetylcysteine (J Biol Chem. 278, 703-11 (2003); J Clin Invest. 92, 1866-74 (1993)). From these findings, it is considered that the expression of adhesion molecules induced upon inflammation can be suppressed by antioxidative action and phase II enzyme induction. ICAM-1 and VCAM-1 relate to conditions of wide varieties of inflammatory diseases such as vasculitis, pulmonary bronchitis, rheumatoid arthritis, osteoarthritis, hepatitis, pancreatitis, dermatitis, esophagitis, ulcerative colitis, Crohn's disease, and conjunctivitis. Further, adhesion molecules on the vascular endothelium, as represented by ICAM-1 and VCAM-1, generally adhere to integrin on the surface of leukocytes such as monocytes by forming a strong bond; however, it is known that when cancer cells are present in the body, metastasis and infiltration occur by the bond with integrin on these cells (Cancer Metastasis Rev. 18, 345-57 (1999)). Therefore, if Nrf2 activation suppresses the expression of these adhesion molecules, it may also lead to the suppression of cancer metastasis and infiltration.

The hop (Humulus luplus) is a native European perennial which belongs to the family Cannabaceae, and its cones (matured female flowers) generally called hops are widely known to be used for adding a bitter taste and aroma to beer and thus have long been ingested by humans. Such bitter taste and aroma come from hop lupulin (yellow granules formed in the root of the inner scales of cones). Hops are also used as a folk medicine and known to have various physiological effects, such as inducing sedation, encouraging sleep, inducing sound sleep, stimulating appetite, soothing stomach, diuretic effect, and antidiabetic effect. Further, it has also been reported that polyphenols derived from hop scales obtained from hop cones, from which the lupulin part is removed, have activities such as inhibiting lipase activity and suppressing body weight gain (Japanese Patent Laid-open Publication No. 2001-321166; Japanese Patent Laid-open Publication No. 2001-131080).

Further, as for humulones and isohumulones which are major bitter-taste components of hops, activity as a PPAR agonist, activity involved in adipocyte differentiation and activity involved in activation of β oxidation enzymes, which suggests such activity, have recently been disclosed. Further, as for this bitter-taste hop component, improvement in insulin resistance, improvement in lipid metabolism such as increase in blood HDL cholesterol and suppression of the accumulation of liver lipid, suppressive effect on body weight gain, preventive effect on fat accumulation, decrease in blood pressure, and improvement in vascular endothelial functions have been also disclosed (WO 03/068205, WO 2004/064818).

However, as far as the present inventors are aware, there are no reports that major bitter-taste hop components, isohumulones, are capable of activating Nrf2, inducing phase II enzymes by the activation, further increasing the amount of the resulting cellular glutathione, and augmenting protective activities against reactive oxygen species or xenobiotic substances. Further, as far as the present inventors are aware, there are no reports on these isohumulones regarding the treatment, prevention, improvement, or amelioration of diseases characterized by oxidative stress, such as cerebral nerve diseases, skin diseases, eye diseases, and asthma.

### SUMMARY OF THE INVENTION

The present inventors have now found that isohumulones, bitter-taste components derived from hops, have Nrf2 activation activity. Further, the present inventors have found that these isohumulones or isomerized hop extract intracellularly induce phase II enzymes, can increase the amount of intracellular glutathione, and suppress cell death caused by reactive oxygen species. In other words, the inventors have found that the isohumulones are capable of accelerating protective activity against oxidative stress and accelerating detoxification of xenobiotic substances and have anti-inflammatory activity. The present invention has been made based on these findings.

An object of the present invention is to provide pharmaceutical agents or foods useful in treating, preventing, improving, or ameliorating chronic diseases which are considered to be caused or exacerbated by cellular damages due to oxidative stress in the body or environmental substances (for example, arteriosclerosis, hypertension, diabetes, cerebral nerve degenerative diseases, skin diseases, eye diseases, asthma, and cancer), or delaying progress of these diseases.

A composition according to the present invention is a composition for use in the treatment, prevention, or amelioration of a disease or condition which is treatable, preventable, or ameliorable by the activation of transcription factor Nrf2, comprising isohumulones or isomerized hop extract as an active ingredient.
A transcription factor Nrf2 activator according to the present invention comprises isohumulones or isomerized hop extract as an active ingredient.

A food according to the present invention is a food for use in the treatment, prevention, or amelioration of a disease or condition which is treatable, preventable, or ameliorable by the activation of transcription factor Nrf2, comprising an effective amount of isohumulones or isomerized hop extract.
A food according to another embodiment of the present invention is a food for use in antioxidation, detoxification, or anti-inflammation, comprising an effective amount of isohumulones or isomerized hop extract.
A food according to yet another embodiment of the present invention is a food for use in the activation of transcription factor Nrf2, comprising an effective amount of isohumulones or isomerized hop extract.

A method according to the present invention for the treatment, prevention, or amelioration of a disease or condition which is treatable, preventable, or ameliorable by the activation of transcription factor Nrf2 comprises the step of administering or feeding a therapeutically effective amount of isohumulones or isomerized hop extract to a mammal.

According to another embodiment of the present invention, there is provided a method of activating the transcription factor Nrf2, comprising the step of administering an effective amount of isohumulones or isomerized hop extract to a mammal.

According to yet another embodiment of the present invention, there is provided use of isohumulones or isomerized hop extract for manufacturing a composition for use in the treatment, prevention, or amelioration of a disease or condition which can be treated, prevented, or ameliorated by the activation of transcription factor Nrf2.
According to still yet another embodiment of the present invention, there is provided use of isohumulones or isomerized hop extract for manufacturing an Nrf2 activating agent.

According to still another embodiment of the present invention, there is provided use of isohumulones or isomerized hop extract for manufacturing an antioxidative agent, a detoxification agent, or an anti-inflammatory agent.

Cerebral nerve degenerative diseases, eye diseases, skin diseases, asthma, cancer, and arteriosclerosis are age-related chronic diseases and their conditions are complicated and associated with an abnormal state of oxidative stress in the body. Their treatment by drugs often takes long period of time and various problems such as incidence of side effects due to the increased amount of dose and the extended administration period cannot be ignored. An active ingredient of the compositions and foods according to the present invention is derived from hops which have been used as food for many years. Therefore, the compositions and foods according to the present invention are advantageous in that they are inexpensive and highly safe and do not cause many side effects even if administered to patients for a long period of time.

### BRIEF DESCRIPTION OF THE DRAWINGS

Fig. 1 shows the induction of QR activity by isomerized hop extract in Example 1 (n=3, mean ± standard deviation).
Fig. 2 shows the induction of QR activity by isohumulones in Example 1 (n=3, mean ± standard deviation).
Fig. 3 shows the induction of QR activity by humulones in Example 1 (n=3, mean ± standard deviation).
Fig. 4 shows the Nrf2 activation by isohumulones in Example 2 (n=2, mean ± standard deviation).
Fig. 5 shows the change in Nrf2 target gene expression by isohumulones in Example 3 (n=2, mean ± standard deviation).
Fig. 6 shows the change in the amount of total glutathione in cells by isohumulones in Example 4 (n=2, mean ± standard deviation).
Fig. 7 shows the effect of isohumulones on cell death by hydrogen peroxide in Example 5 (n=2, mean ± standard deviation). Black bars and white bars show hydrogen peroxide concentrations of 0.8 mM and 1.0 mM, respectively.
Fig. 8 shows the suppression of TNF-α production by isomerized hop extract or isohumulones in Example 6 (n=3, mean ± standard deviation).
Fig. 9 shows the suppression of ICAM-1 and VCAM-1 expression by isomerized hop extract in Example 8.
Fig. 10 shows the change in liver gene expression by oral administration of isomerized hop extract in Example 9 (n=5, mean ± standard deviation).

### DETAILED DESCRIPTION OF THE INVENTION

### Active ingredients and methods for their production Isohumulones

In the present invention, isohumulones are not particularly limited as long as they are known as isohumulone and its related compounds and include isohumulone, isoadhumulone, isocohumulone, isoposthumulone, isoprehumulone, tetrahydroisohumulone, alloisohumulone, paraisohumulone, humulinic acid, hexahydroisohumulone, antiisohumulone, and hulupone.

Further in the present invention, isohumulones also include pharmaceutically acceptable salts and solvates of the abovementioned isohumulone compounds, as well as the compounds themselves. Examples of such pharmaceutically acceptable salts are acid addition salts. Examples of the acid addition salts include salts of inorganic acids such as hydrochloric acid, hydrobromic acid, and sulfuric acid; and salts of organic acids such as citric acid, oxalic acid, malic acid, tartaric acid, fumaric acid, maleic acid, methanesulfonic acid, and salicylic acid. Further, compounds having a carboxyl group can be salts with metals such as sodium, potassium, calcium, magnesium, and aluminium or salts with amino acids such as lysine. Further, examples of the pharmaceutically acceptable solvates include hydrates, alcoholates (for example, methanolates and ethanolates), and etherates.

According to a preferred embodiment of the present invention, isohumulones can be selected preferably from the group consisting of isohumulone, isoadhumulone, isocohumulone, and combinations thereof.

Further, isohumulones can be cis-trans isomers derived from substituents, alkenyl groups. The present invention includes both cis isomers and trans isomers and mixtures thereof. According to a preferred embodiment, isohumulones are trans-form isohumulones.

Isohumulones herein can be those commercially available. An active ingredient in the present invention can also be produced according to known methods; for example, it can be synthesized by the method described in Developments in Food Science 27, CHEMISTRY AND ANALYSIS OF HOP AND BEER BITTER ACIDS, M. Verzele, ELSEVIER. An active ingredient according to the present invention can also be isolated and purified from a hop extract or isomerized hop extract obtained by the method described below.

### Isomerized hop extract

An active ingredient according to the present invention can be a product prepared from a natural material such as hops. An active ingredient according to the present invention is present, for example, in hop cones, hop extract, or isomerized products thereof and can be fractionated from these materials using various chromatographic techniques (see, for example, "The components of Brewing Product," December 10, 1999, published by Brewing Society of Japan).

Further, in the present invention, isomerized hop extract can be obtained by isomerizing hop extract derived from hop lupulin. The hop is a perennial plant which belongs to the family Cannabaceae, and hops are its cones (matured unpollinated female flowers). Hop lupulin is a raw material for beer brewing and is used to impart a bitter taste and aroma to beer. In the beer brewing process, humulones (for example, humulone, cohumulone, adhumulone, posthumulone, and prehumulone) in hops are isomerized to isohumulones (for example, isohumulone, isocohumulone, isoadhumulone, isoposthumulone, and isoprehumulone) to impart a characteristic taste and aroma to beer.

Hop extract can be prepared by subjecting cones or a pressed product thereof, as it is or after crushing, to an extraction process. The extraction can be carried out, for example, by the extraction method using ethanol solvent which is used for the preparation of hop extract for the beer brewing and the supercritical carbon dioxide extraction method. In particular, the supercritical carbon dioxide extraction is characterized in that the resulting product contains a low concentration of polyphenol components and highly concentrated bitter component and essential oil component. Further, hop extraction can be carried out using other generally used methods, including a method in which hop cones, crushed products thereof, or the like are submersed in a cold or warmed solvent; a method in which extraction is carried out with heating and stirring and then the extract is obtained by filtration; and a percolation method. After removing solids by filtration or centrifugation if necessary, the resulting extract can be used as it is or after removing the solvent by distillation and partially concentrating or drying, depending on the mode of use. Further, after concentrating or drying, the extract can be washed and purified with an insoluble solvent or further dissolved or suspended in an appropriate solvent, for use. Further in the present invention, for example, the solvent extract obtained as described above can be dried using general means such as drying under reduced pressure and freeze drying to obtain a dried hop extract for use.

Examples of the solvent to be used for the abovementioned extraction include water; lower alcohols having 1-4 carbon atoms, such as methanol, ethanol, propanol, and butanol; lower alkyl esters such as ethyl acetate ester; glycols such as ethylene glycol, butylene glycol, propylene glycol, and glycerin; polar solvents such as ethyl ether, acetone, and acetic acid; hydrocarbons such as benzene and hexane; non-polar solvents such as ethers, e.g., ethyl ethers and petroleum ethers; and other known organic solvents. These solvents can be used in combination of two or more kinds.

Further, if necessary, insolubles can be removed by filtration, extracts can be concentrated by reduced pressure and the like, or the solvent can be dried to solid. Further, crushed cones can preferably be subjected to the supercritical carbon dioxide extraction or the liquid carbon dioxide gas extraction.
The crude extracts thus prepared contain humulones and their isomers, isohumulones. Isohumulones to be used in the present invention can be isolated and purified from these crude extracts using a conventional method. Further, these crude extracts can be used as they are as isomerized hop extract in the present invention, depending on the isohumulone content or the like.

In order to obtain a product having a higher isohumulone content, it is preferable to further isomerize these crude extracts by heating in the presence of alkali or magnesium oxide. By the isomerization, humulones in the hop extracts are completely converted into isohumulones.

The process of isomerization will be explained in more detail as follows. Hop extract is dissolved in an alcohol solvent such as ethanol, after which weak alkaline water is added thereto and then the hop extract is heat-isomerized by flowing back with heat (for example, at about 92-93°C) to obtain isomerized hop extract. The isomerized hop extract thus obtained can be concentrated or purified by a known method (for example, filtration, concentration under reduced pressure, or lyophilization), if necessary. From the viewpoint of safety, the weak alkaline water (for example, pH 8.5 to 9.5) to be used in the abovementioned isomerization can be, for example, commercially available water (e.g., bottled water) such as alkaline ion water for drink. Use of commercially available drinking water is highly safe since it has a significantly long history of use. Further, since the mode of reaction in production by heat-isomerization in the boiling wort process in brewing beer is substantially equivalent to the abovementioned isomerization process, the safety is high from the viewpoint of providing foods and drinks.

In the present invention, the isomerized hop extract obtained as mentioned above can be used directly for manufacturing compositions, foods, and the like; however, it is preferable to use a fraction containing an active ingredient at a higher concentration.

Hop extracts and isomerized extracts extracted by various methods are commercially available as beer additives. Accordingly, in the present invention, these commercially available hop extracts or isomerized hop extracts can be used as they are or after being subjected to a further isomerization process if necessary. Examples of the commercially available isomerized hop extracts to be used include a hop extract in which humulones and lupulones are primarily extracted from crushed hop cones using the supercritical carbon dioxide extraction method (for example, CO2 Pure Resin Extract (Hopsteiner)), an isomerized extract of a carbon dioxide extract of crushed hop cones (for example, Isomerized Kettle Extract (SS. Steiner), primarily consisting of isohumulones and lupulones), and a water-soluble extract which is prepared by isomerizing a carbon dioxide extract of crushed hop cones and then converting the product into a potassium salt to obtain a low viscous fluid (for example, ISOHOPCO2N (English Hop Products) and ISOHOPR (Botanix), primarily consisting of isohumulones).
Further, a fraction containing isohumulones at a higher concentration than these extracts can be obtained by concentration or any of the abovementioned methods.

According to a preferred embodiment of the present invention, an active ingredient in a composition, Nrf2 activator, or food of the present invention can be isomerized hop extract.

### Use

An active ingredient according the present invention, isohumulones or isomerized hop extract, has a function to activate the transcription factor Nrf2 (Examples 1, 2, and 3).

It is known that the transcription factor Nrf2 is deeply involved in protective activities of the body against oxidative stress or xenobiotic substances. It has been reported that Nrf2 activators can treat, prevent, improve, or ameliorate diseases or conditions characterized by these activities, or delay their progress. Examples of such diseases or conditions with relevant reference are as follows: hypertension (Proc Natl Acad Sci USA. 101, 7094-9 (2004)), inflammations (Proc Natl Acad Sci USA. 101, 7094-9 (2004)), cerebral nerve degenerative diseases (J Biol Chem. 277, 388-94 (2002); J Neurochem. 71, 69-77 (1998); Neurosci Lett. 273, 109-12 (1999)), eye diseases (Proc Natl Acad Sci USA. 101, 10446-51 (2004); Mol Vis. 9, 223-30 (2003); Invest Ophthalmol Vis Sci. 43, 1162-7 (2002)), skin diseases (J Clin Invest. 113, 65-73 (2004)), asthma (J Immunol. 173, 3467-81 (2004)), and carcinogenesis (Proc Natl Acad Sci USA. 89, 2399-403 (1994); Carcinogenesis. 21, 2287-91 (2000); Proc Natl Acad Sci USA. 98, 11551-6 (2001)). Further, Nrf2 activators are known to be involved in the detoxification of xenobiotic substances and in the treatment, prevention, or amelioration of chronic inflammations as mentioned above in the prior art section. These actions are believed to be due to the augmentation in protective activities against oxidative stress and xenobiotic substances by the increase in the expression of a series of phase II enzymes and the amount of glutathione associated with Nrf2 activation.

An active ingredient according to the present invention increased quinone reductase activity (QR activity) which is a typical enzyme activity increased by Nrf2 activation (Example 1). An active ingredient according to the present invention showed Nrf2 activation in the reporter assay regarding an ARE (antioxidant responsive element) sequence (Example 2). Further, an active ingredient according to the present invention increased the expression of a series of phase II enzymes whose expression is augmented by Nrf2 activation (Example 3). Further, an active ingredient according to the present invention increased the amount of glutathione in cells (Example 4) and suppressed cell death by reactive oxygen species (Example 5). Accordingly, an active ingredient according to the present invention can be said to have a function to activate the transcription factor Nrf2.

An active ingredient according to the present invention suppressed TNF-α production from macrophages by an inflammation inducing substance (Example 6). Further, the administration of isomerized hop extract significantly decreased the amounts of sICAM-1 and sVCAM-1 which are considered to be markers for suggesting inflammatory conditions (Example 7). Further, isomerized hop extract markedly suppressed the expression of ICAM-1 and VCAM-1 on the vascular endothelium by TNF-α stimulation (Example 8). Accordingly, isohumulones are shown to markedly suppress the expression of adhesion molecules on the vascular endothelium in the early inflammatory process and suppress adhesion and infiltration of leukocytes such as monocytes, in the progress of arteriosclerosis under high fat dieting conditions. Namely, isohumulones can be said to be able to treat, prevent, or ameliorate the vascular inflammation which occurs in the early stage of arteriosclerosis. Further, isohumulones can be said to prevent or suppress metastasis or infiltration of cancer since they can suppress the expression of adhesion molecules on the vascular endothelium, ICAM-1 and VCAM-1.

Further, an active ingredient according to the present invention significantly increased expression of typical liver phase II enzymes GSTM1, NQO1, and GCLC in an in vivo oral administration test (Example 9). This result shows that also in the case where an active ingredient according to the present invention is orally administered, Nrf2 is activated in the body and the expression of a series of genes for so-called phase II enzymes is augmented. GSTM1 and NQO1 are enzymes involved in detoxification and GCLC is an enzyme directing the synthesis of an antioxidant, glutathione, so that the active ingredient of the present invention can be said to accelerate the detoxification and antioxidative activities. Further, in the abovementioned in vivo experiment, an active ingredient according to the present invention did not increase the expression of phase I enzymes which are involved in the activation of carcinogenic substances represented by Cyp1A1 and Cyp1A2 (Example 9). From these results, it can be said that an active ingredient of the present invention selectively increases the expression of phase II enzymes in the metabolism of the detoxification system.

Therefore, an active ingredient according to the present invention can be used for the treatment, prevention, or amelioration of a disease or condition which is treatable, preventable, or ameliorable by the activation of transcription factor Nrf2. In the present invention, the expression "treatment, prevention, or amelioration" of a disease or condition implies control, delay in progress, amelioration, prevention of onset, prevention of recurrence, or suppression of a disease or condition.

Examples of the disease and condition which is treatable, preventable, or ameliorable by the activation of transcription factor Nrf2 include oxidative stress, detoxification of xenobiotic substances, chronic inflammatory diseases, and diseases and conditions related thereto. More specifically, examples of the oxidative stress or diseases and conditions related thereto include cerebral nerve degenerative diseases, eye diseases, skin diseases, asthma, cancer, arteriosclerosis and diseases or conditions related thereto. Examples of the cerebral nerve degenerative diseases include Alzheimer's disease, Parkinson's disease, and amyotrophic lateral sclerosis. Further, examples of the eye diseases include age-related macular degeneration, cataracts, light retinopathy, and retinopathy of prematurity. Specific examples of the chronic inflammatory diseases include vasculitis, pulmonary bronchitis, rheumatoid arthritis, osteoarthritis, hepatitis, pancreatitis, dermatitis, esophagitis, ulcerative colitis, Crohn's disease, and conjunctivitis.

Further, an active ingredient according to the present invention can be used as an active ingredient of a transcription factor Nrf2 activator. An active ingredient according to the present invention can be used, for example, for increasing the amount of intracellular glutathione, accelerating the expression of phase II enzymes, and suppressing cell death by reactive oxygen species. Further, an active ingredient according to the present invention can also be used for the suppression of blood pressure increase, improvement of vascular endothelial functions, prevention of carcinogenesis, treatment of cancer, diabetes, and arteriosclerosis, and the like. Further, an active ingredient according to the present invention can be used for preventing or suppressing metastasis or infiltration of cancer cells and for treating, preventing, or ameliorating inflammation of the arterial wall which leads to arteriosclerosis. Here, since the preventive or suppressive effect on metastasis or infiltration of cancer cells works through a mechanism different from that for prevention of carcinogenesis, it should be evident also for those skilled in the art that having a preventive effect on carcinogenesis does not necessarily mean having a preventive or suppressive effect on metastasis or infiltration of cancer cells.
Namely, an active ingredient according to the present invention can be used also as an active ingredient of antioxidants, detoxification agents, or anti-inflammatory agents.

According to the present invention, there is provided a method of treating, preventing, or ameliorating a disease or condition which is treatable, preventable, or ameliorable by the activation of transcription factor Nrf2, comprising the step of administering or feeding a therapeutically effective amount of isohumulones or isomerized hop extract to a mammal.
The term "therapeutically effective amount" means an effective amount necessary at least to exhibit desired effects such as therapeutic, preventive and ameliorating effects.

According to the present invention, there is provided a method of activating the transcription factor Nrf2, comprising the step of administering a therapeutically effective amount of isohumulones or isomerized hop extract to a mammal.

According to the present invention, there is provided use of an active ingredient according to the present invention for manufacturing an Nrf2 activator.

Further, according to the present invention, there is provided use of isohumulones or isomerized hop extract for manufacturing an antioxidant, detoxification agent, or anti-inflammatory agent.

Further, according to the present invention, there is provided use of isohumulones or isomerized hop extract for manufacturing a composition for use in treating, preventing, or ameliorating a disease or condition which is treatable, preventable, or ameliorable by the activation of transcription factor Nrf2.

### Compositions and foods

A composition according to the present invention comprises isohumulones or isomerized hop extract as an active ingredient as mentioned above.
The expression "comprising as an active ingredient" herein naturally means that physiologically acceptable carriers can be included according to a desired product form and also means that other auxiliary components usable together can be included. Namely, a composition according to the present invention can be manufactured by mixing an active ingredient, i.e., isohumulones or isomerized hop extract, with physiologically acceptable carriers, excipients, binding agents, diluents, and the like. A composition according to the present invention can be administered or fed orally or non-orally. Examples of oral formulations include food products, granules, dispersible powders, tablets (including sugar-coated tablets), pills, capsules, syrups, emulsions, and suspensions. Examples of non-oral formulations include injections (for example, subcutaneous injections, intravenous injections, intramuscular injections, and intraperitoneal injections), intravenous drips, preparations for external use (e.g., nasal formulations, percutaneous agents, and ointments), and suppositories (e.g., rectal suppositories and vaginal suppositories). These pharmaceutical preparations can be formulated by a method generally used in this field using pharmaceutically acceptable carriers (e.g., excipients and additives). Examples of the pharmaceutically acceptable excipients and additives include carriers, binding agents, flavoring agents, buffers, thickening agents, coloring agents, stabilizers, emulsifying agents, dispersing agents, suspending agents, and preservatives. Examples of the pharmaceutically acceptable carriers include magnesium carbonate, magnesium stearate, talc, sucrose, lactose, pectin, dextrin, starch, gelatin, tragacanth, methylcellulose, sodium carboxymethylcellulose, low-melting wax, and cacao butter.

Examples of other auxiliary components usable together include amino acids such as cysteine, cystine, glutamine, and glycine, vitamins (e.g., vitamin D and vitamin K₂), and antibiotics.

Pharmaceutical preparations can be manufactured, for example, as follows.
Oral formulations can be manufactured by adding excipients (e.g., lactose, white sugar, starch, and mannitol), disintegrating agents (e.g., calcium carbonate and calcium carboxymethylcellulose), binding agents (e.g., pregelatinized starch, gum arabic, and carboxymethylcellulose, polyvinylpyrrolidone, and hydroxypropylcellulose), lubricating agents (e.g., talc, magnesium stearate, and polyethylene glycol 6000), or the like, to an active ingredient, compressing the admixture into an appropriate form, and if necessary, coating for the purpose of taste masking, enteric coating, or durability, using a known method. Examples of coating agents to be used include ethylcellulose, hydroxymethylcellulose, polyoxyethylene glycol, cellulose acetate phthalate, hydroxypropylmethylcellulose phthalate, and Eudragit (a methacrylic acid-acrylic acid copolymer; Roehm, Germany).

Formulations for injection can be manufactured by dissolving, suspending, or emulsifying an active ingredient in an aqueous solvent (e.g., distilled water, physiological saline, and Ringer's solution) or an oleaginous medium (e.g., vegetable oils such as olive oil, sesame oil, cotton seed oil, and corn oil, and propylene glycol) together with dispersing agents (e.g., Tween 80 (Atlas Powder, USA), HCO 60 (Nikko Chemicals), polyethylene glycol, carboxymethylcellulose, and sodium alginate), preservatives (e.g., methylparabene, propylparabene, benzylalcohol, chlorobutanol, and phenol), osmosis equilibrating agents (e.g., sodium chloride, glycerin, sorbitol, glucose, and invert sugars), or the like. If desired, additives such as solubilizing agents (e.g., sodium salicylate and sodium acetate), stabilizing agents (e.g., human serum albumin), and analgesic agents (e.g., benzalkonium chloride and procaine hydrochloride) may be added.

Pharmaceutical preparations for external use can be manufactured by formulating an active ingredient into a solid, semi-solid or liquid composition. For example, the abovementioned solid composition can be manufactured by formulating an active ingredient as it is or by admixing with excipients (e.g., lactose, mannitol, starch, microcrystal cellulose, and white sugar), thickening agents (e.g., natural gums, cellulose derivatives, and acrylic acid polymers), and the like, into a powder. The abovementioned liquid composition can be manufactured in almost the same manner as described for formulations for injection. The semi-solid composition is preferably an aqueous or oleaginous gel or ointment. Further, these compositions can all contain pH controlling agents (e.g., carbonic acid, phosphoric acid, citric acid, hydrochloric acid, and sodium hydroxide), preservatives (e.g., paraoxybenzoic acid esters, chlorobutanol, and benzalkonium chloride), and the like.

Suppositories can be manufactured by formulating an active ingredient into an oleaginous or aqueous solid, semi-solid, or liquid composition. Examples of the oleaginous base to be used for such composition include higher fatty acid glycerides (e.g., cacao oil and Witepsols (Dynamite Nobel)), medium fatty acids (e.g., Miglyols (Dynamite Nobel)), and vegetable oils (e.g., sesame oil, soybean oil, and cotton seed oil). Examples of the aqueous base include polyethylene glycols and propylene glycols. Further, examples of the aqueous gel base include natural gums, cellulose derivatives, vinyl polymers, and acrylic acid polymers.

Upon formulation, one or more kinds of medically effective active ingredients other than an active ingredient according to the present invention can be further admixed. Further, upon administering an active ingredient according to the present invention, one or more kinds of medically effective active ingredient other than an active ingredient according to the present invention can be combined for administration. Examples of such other active ingredients include, but are not limited to, Nrf2 activators, antioxidants, detoxification agents, and anti-inflammatory agents.

Examples of the Nrf2 activators include sulforaphane, avicins, 15dPGJ₂, xanthohumol, curcumin, carnosol, zerumbone, isothiocyanate, α-lipoic acid, oltipraz (4-methyl-5-[2-pyrazinyl]-1,2-dithiole-3-thione), 1,2-dithiole-3-thione, and 2,3-butyl-4-hydroxyanisole. Examples of the antioxidants include vitamin C, vitamin E, carotenoids, retinoids, polyphenols, flavonoids, lignan, selenium, butylated hydroxyanisole, ethylene diamine tetra-acetate, calcium disodium, acetylcysteine, probucol, and tempol. Examples of the detoxification agents include dimethyl caprol, glutathione, acetylcysteine, methionine, sodium hydrogen carbonate, deferoxamine mesylate, calcium disodium edetate, trientine hydrochloride, penicillamine, and pharmaceutical charcoal. The anti-inflammatory agents include steroidal anti-inflammatory agents and non-steroidal anti-inflammatory agents. Examples of the steroidal anti-inflammatory agents include cortisone acetate, hydrocortisone, paramethasone acetate, prednisolone, prednisolone, methylprednine, dexamethasone, triamcinolone, and betamethasone. Examples of the non-steroidal anti-inflammatory agents include salicylic acid non-steroidal anti-inflammatory agents such as aspirin, diflunisal, aspirin + ascorbic acid, and aspirin dialuminate; aryl acid non-steroidal anti-inflammatory agents such as diclofenac sodium, sulindac, fenbufen, indomethacin, indomethacin farnesyl, acemetacin, proglumetacin maleate, anfenac sodium, nabmeton, mofezolac, and etodorag; fenamic acid non-steroidal anti-inflammatory agents such as mefenamic acid, flufenamic acid aluminum, tolfenamic acid, and floctafenine; propionic acid non-steroidal anti-inflammatory agents such as ibuprofen, flurbiprofen, ketoprofen, naproxen, pranoprofen, fenoprofen calcium, thiaprofen, oxaprozin, loxoprofen sodium, alminoprofen, and zaltoprofen; oxicam non-steroidal anti-inflammatory agents such as piroxicam, ampiroxicam, tenoxicam, lornoxicam, and meloxicam; and basic non-steroidal anti-inflammatory agents such as tiaramide hydrochloride, epirizole, and emorfazone.

The dosage form of the pharmaceutical preparation can be appropriately selected according to a target disease or condition. For example, for use in treating, preventing, or ameliorating a skin disease, a composition according to the present invention can be applied on a target skin part as an agent for external use.

A composition and an activating agent according to the present invention are intended to be applied not only to pharmaceutical products but also to food products. Accordingly, upon applying a composition and an activating agent of the present invention to food products, the description for food products below can be referred to.

A food according to the present invention comprises an effective amount of an active ingredient according to the present invention.
The expression "comprising an effective amount of an active ingredient" herein means that an individual food or drink contains the active ingredient so that its ingestion in an ordinary amount results in the ingestion of the active ingredient in the amount in the range described below. In a food according to the present invention, an active ingredient according to the present invention can be blended into the food as it is or in the form of the abovementioned composition or activating agent. More specifically, a food according to the present invention can be a food prepared using an active ingredient or the abovementioned crushed hop preparation or extract thereof as it is or after isomerization, a food prepared by further admixing various proteins, sugars, fats, trace elements, vitamins, and the like, a food further prepared into the form of liquid, semi-liquid, solid, or paste, or a food adding the above to a general food or drink.

The "food" used in the present invention is not particularly limited as long as it is non-drug and can be ingested by a mammal, including those in liquid, semi-solid and solid forms. Accordingly, the food also includes drinks and the like.

The term "food" used in the present invention also includes those categorized as health foods, functional foods, foods for specified health use, nutrition supplement foods, foods claiming to reduce disease risk, and foods for patients. Further, when used for non-human mammals, the term "food" can also include feeds.

The food can also be in the form of ordinary food or nutrition supplement food.

An active ingredient according to the present invention has effects to prevent or ameliorate chronic diseases associated with cellular damages due to oxidative stress or environmental pollutants (for example, cerebral nerve degenerative diseases, eye diseases, skin diseases, and asthma), or to suppress their progress. Accordingly, it is possible to provide a food product which simultaneously functions in preventing and ameliorating various chronic diseases associated with cellular damages due to oxidative stress or environmental pollutants by blending an active ingredient according to the present invention into an everyday food, health food, functional food, or supplement (for example, a food containing one or more kinds of minerals such as calcium and magnesium and vitamins such as vitamin K).

According to the present invention, as mentioned above, there is provided a food for use in treating, preventing or ameliorating a disease or condition which is treatable, preventable, or ameliorable by the activation of transcription factor Nrf2, comprising an effective amount of isohumulones or isomerized hop extract.
According to the present invention, there is also provided a food for antioxidation, detoxification, or anti-inflammation, comprising an effective amount of isohumulones or isomerized hop extract.
Further, according to the present invention, there is provided a food for use in the activation of transcription factor Nrf2, comprising an effective amount of isohumulones or isomerized hop extract.

According to another embodiment of the present invention, there is provided a food which carries an indication for its function to treat, prevent, or ameliorate a disease or condition which is treatable, preventable, or ameliorable by the activation of transcription factor Nrf2, comprising an effective amount of isohumulones or isomerized hop extract.
According to yet another embodiment of the present invention, there is provided a food which carries an indication for its function in antioxidation, detoxification, or anti-inflammation, comprising an effective amount of isohumulones or isomerized hop extract.
According to still another embodiment of the present invention, there is provided a food which carries an indication for its function in the activation of transcription factor Nrf2, comprising an effective amount of isohumulones or isomerized hop extract.

Here the indication for the function which the food product carries is placed onto the product itself, a container, a package, an instruction, a package insert, or an advertisement.

Therefore, a food according to the present invention can be provided as a food appropriate for a consumer who expects functions to ameliorate or alleviate conditions related to oxidative stress in the body, entry of xenobiotic substances into the body, or inflammation; a food appropriate for a consumer who expects functions to ameliorate or alleviate dementia; a food appropriate for a consumer who expects functions to ameliorate or alleviate conditions associated with aging of the eye or skin; or a food appropriate for a consumer who expects functions to ameliorate or alleviate asthma or conditions associated with it; namely as a food for specified health use. The food for specified health use herein means a food which is regulated by certain laws in individual countries from the viewpoint of public health when the food is, for example, manufactured or sold for the purpose of, for example, preventing, ameliorating, or alleviating a disease or condition which associates with cellular damages due to oxidative stress or environmental pollutants. Such food can be a food indicating a potential for reducing disease risk, namely a food carrying an indication for a disease risk reduction. The indication for a disease risk reduction herein is an indication for a food having potential to reduce disease risk, which can be an indication provided or approved based on or referring to the standard defined by the FAO/WHO joint committee on food standardization (Codex Committee).

Specific examples of the food according to the present invention include drinks and foods containing carbohydrate such as rice products, noodles, breads, and pastas; various confectionaries including Western sweets such as cookies and cakes, Japanese sweets such as buns with a filling and steamed adzuki-bean pastes, candies, chewing gums, and chilled or icy sweets such as yogurt and puddings; various drinks such as juices, soft drinks, milk drinks, tea drinks, functional drinks, nutrient supplement drinks, and non-alcoholic beer; alcoholic drinks such as beer and low-malt beer; processed foods with eggs; and processed foods (including delicacies) using seafood (e.g., squid, octopus, shellfish, and eel), or meat (including entrails such as liver).

According to a preferred embodiment of the present invention, examples of the food for admixing are drinks (e.g., tea drinks and milk drinks) and yogurt.

According to a more preferred embodiment of the present invention, the drink is a non-alcoholic drink.

The term "tea drinks" as used herein refers to drinks which are made by infusing leaves of the tea plant belonging to an evergreen shrub of the Theacae family (tea leaves), leaves of plants other than the tea plant, or grains, and includes fermented tea, semi-fermented tea and non-fermented tea. Specific examples of the tea drinks include Japanese tea (e.g., green tea and roasted barley tea), black tea, herbal tea (e.g., jasmine tea), Chinese tea (e.g., Chinese green tea and Oolong tea), and roasted tea.

The term "milk drinks" as used herein refers to raw milk, cow's milk, and the like, or drinks which are manufactured using a food product using them as a major raw material, and includes those which use cow's milk or the like as a raw material as well as those which use processed milks such as nutrient fortified milk, flavored milk, and sweetened hydrolyzed milk, as a raw material.

Further, "yogurt" as used herein includes hard yogurt, soft yogurt, and drink-type yogurt as well as processed yogurt products which use yogurt as a raw material.

Generally foods derived from hops such as beer, non-alcoholic beer, and low-malt beer occasionally already contain a certain amount of an active ingredient of the present invention. Namely, such known foods containing isohumulones are not foods to which a hop component is added but foods manufactured through a process in which a hop component is isomerized. Such foods can be used as they are as foods according to the present invention; however, by admixing isohumulones, an extract containing them, or isomerized hop extract to such foods, it is possible to further fortify desired effects or to reduce the effective amount for ingestion. The expression "to fortify" as used herein means to admix an active ingredient or an extract according to the present invention so that the amount of the active ingredient originally contained in the food exceeds the amount necessary to exhibit expected functions.

Beer is a known example of food containing isohumulones. For example, the isohumulone content of beer is about 10 to 50 mg/L.

In a food according to the present invention, the use of isomerized hop extract is considered to be more advantageous than the use of purified isohumulones in terms of cost effectiveness. However, if cost is not a concern, purified isohumulones can be added during the food manufacturing process or to the final product, or in the case where the manufacturing process comprises an isomerization step, purified humulones can be added prior to the step.

Examples of the form of the health foods or functional foods ingested as a supplement, to which an active ingredient or a hop extract of the present invention is admixed, include drinks such as juice and tea, jelly, capsules, granules, pills, and paste. A health food such as a supplement or functional food which has Nrf2 activation activity, anti-oxidative activity, detoxification activity, anti-inflammatory activity, or a function to ameliorate or alleviate conditions associated with these activities can be provided by processing an active ingredient or a hop extract of the present invention alone or in combination with other components (for example, plant materials) into the form of drinks, jelly, capsules, granules, pills, paste and the like. In particular, a function of preventing, ameliorating, or alleviating conditions which can be prevented or ameliorated by Nrf2 activation can be further fortified by combining with other components known to have antioxidative, detoxification, or anti-inflammatory activity, in addition to an active ingredient according to the present invention. The other components known to have antioxidative activity, detoxification activity, or anti-inflammatory activity can be selected from known components. Examples of the components having antioxidative activity include vitamin C, vitamin E, carotenoids, retinoids, polyphenols, flavonoids, lignan, selenium, butylhydroxyanisole, and calcium disodium ethylenediaminetetraacetate. Examples of the components having detoxification activity include glutathione, methionine, and sodium hydrogen carbonate. Examples of the components having anti-inflammatory activity include allicin, capsicin, bromelain, and allyl sulfide. These components are contained in plant materials; exemplary plant materials containing allicin are onions, Chinese chive, garlic, garlic chive, and green onions; an exemplary plant material containing capsicin is red pepper; an exemplary plant material containing bromelain is pineapple; and exemplary plant materials containing allyl sulfide are asatsuki (baby scallion, Allium schoenoprasum L. var. foliosum Regel), garlic, shallot, Welsh onion, green onion, garlic chive, and rakkyo onion. Further, a multi-functional food can be provided by the combination with other components which exhibit functions other than those according to the present invention or with other functional foods.

In manufacturing drinks provided in the present invention (including drink-type health foods and functional foods), sugars, flavors, juice, food additives, and the like which are used in formulation of ordinary drink products can be appropriately added. Further, in manufacturing drinks, manufacturing technologies known in the art, for example, those in "New Edition Soft Drinks" (Korin Publishing Co., Ltd.) can be referred to.

According to a preferred embodiment of the present invention, isohumulones or isomerized hop extract can be admixed into a food product which originally contains isohumulones or isomerized hop extract (e.g., beer) for the purpose of fortifying or augmenting Nrf2 activating activity, antioxidative activity, detoxification activity, anti-inflammatory activity, or a function of ameliorating or alleviating conditions related to these activities.

When an active ingredient or an extract according to the present invention is used as a food as it is or by admixing with a raw material of a general food, it is desirable to prevent the food from being affected by bitterness of the active ingredient by limiting the amount of use or manipulatively masking the bitterness. For example, the bitterness can be masked by capsulating the active ingredient or the extract or coating it with an appropriate coating agent. Exemplary capsule forms are gelatin capsules and pullulan capsules, and an exemplary coated form is a sugar-coated tablet.

A food according to the present invention can be made into various forms and can be manufactured in accordance with known technology for manufacturing pharmaceutical products. In such cases, it can be manufactured using the carriers or additives for manufacturing pharmaceutical products as mentioned above in the section for manufacturing therapeutic agents according to the present invention, more specifically, using the carriers or additives mentioned in the section for oral formulations.

Since isohumulones or isomerized hop extract, an active ingredient of the present invention, are components derived from hops that have been ingested by humans for many years as a food or drink such as beer, they are low in toxicity and can be used safely for mammals (e.g., humans, mice, rats, rabbits, canines, cats, cattle, horses, pigs, and monkeys).

When a composition and a food according to the present invention are administered or taken, the amount of administration or intake of an active ingredient according to the present invention can be determined depending on the recipient, recipient's age and body weight, symptoms, the time of administration, the type of dosage form, the route of administration, the combination with other medicines, and the like. For example, when administered as a medicine, an active ingredient according to the present invention can be administered to an adult at a dose ranging from 0.005 to 50 mg/kg body weight (preferably 0.05 to 10 mg/kg body weight) as the amount of isohumulones, in a single dose or in several divided doses daily. More specifically, for example, when orally administered as a medicine, an active ingredient according to the present invention can be administered to an adult at a dose ranging from 0.05 to 50 mg/kg body weight (preferably 0.5 to 10 mg/kg body weight) or non-orally at a dose ranging from 0.005 to 10 mg/kg body weight (preferably 0.05 to 2 mg/kg body weight) as the amount of isohumulones, in a single dose or in several divided doses daily. Appropriate dosages of medicines having other functions to be used in combination with an active ingredient according to the present invention can be determined based on their individual dosage for clinical use. Further, when taken as a food, an active ingredient according to the present invention can be admixed in the food so that the amount of its daily ingestion for an adult ranges from 3 to 3000 mg, preferably from 30 to 600 mg. The amount of administration or intake can be calculated and expressed, if necessary, as the amount of isohumulones for daily administration or intake for an adult having a body weight of 60 kg assuming that the adult body weight is 60 kg.

### EXAMPLES

The present invention is further illustrated by the following examples that are not intended as a limitation of the invention.

### Example 1: Induction of quinone reductase (QR) activity by isohumulones

It has been known that quinone reductase (QR) activity is increased by increasing the expression of NAD(P)H:quinone oxidoreductase (NQO1) by Nrf2 activation (Proc Natl Acad Sci USA. 101, 10446-51 (2004)). Accordingly, isohumulones were allowed to act on mouse hepatoma Hepa1c1c7 cells (available from Dainippon Pharmaceutical Co., Ltd.) to examine whether the QR activity was induced.
The QR activity was measured in accordance with the method described in Analytical Biochemistry 169, 328-336 (1988).

A mouse hepatoma cell line Hepalclc7 was seeded on a 96-well plate (7-10 × 10³ cells/well), 50 µl each of an α-MEM medium (Invitrogen) supplemented with 10% fetal bovine serum (HyClone) and penicillin-streptomycin (100 units/ml and 100 µg/ml, respectively; Sigma) was added, and the plate was incubated at 37°C in an atmosphere of 5% CO₂ overnight. Next, the culture medium was replaced with a medium containing isomerized hop extract individually at a concentration of 1.9, 5.6, 16.7, or 50 µg/ml, or with a medium containing humulone (H), cohumulone (cH), adhumulone (aH), isohumulone (IH), isocohumulone (IcH), or isoadhumulone (IaH) individually at a concentration of 0.6, 1.7, 5, and 15 µM and the plate was further incubated for 48 hours. Then, the cells in each well were broken using 0.08% digitonin and a 2 mM EDTA solution and absorption at 610 nm was measured by a menadione-formazan system using a microplate reader (Power Wave 200, Bio-Tek Instruments).
The QR inducing activity was calculated as a relative activity setting the activity of cells without addition to be 1 (n=3, mean ± standard variation). Sulforaphane (Sul, available from Sigma) was used as a positive control.

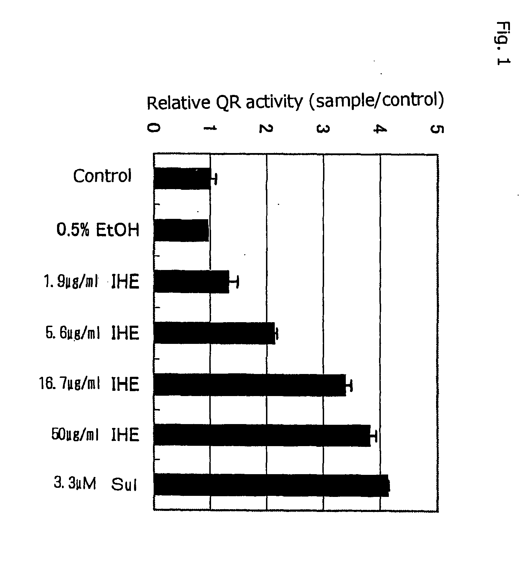

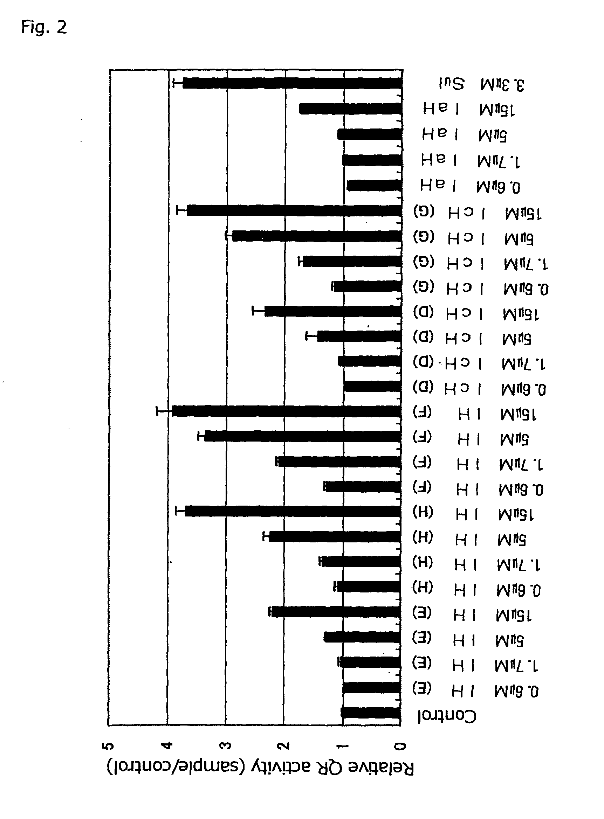

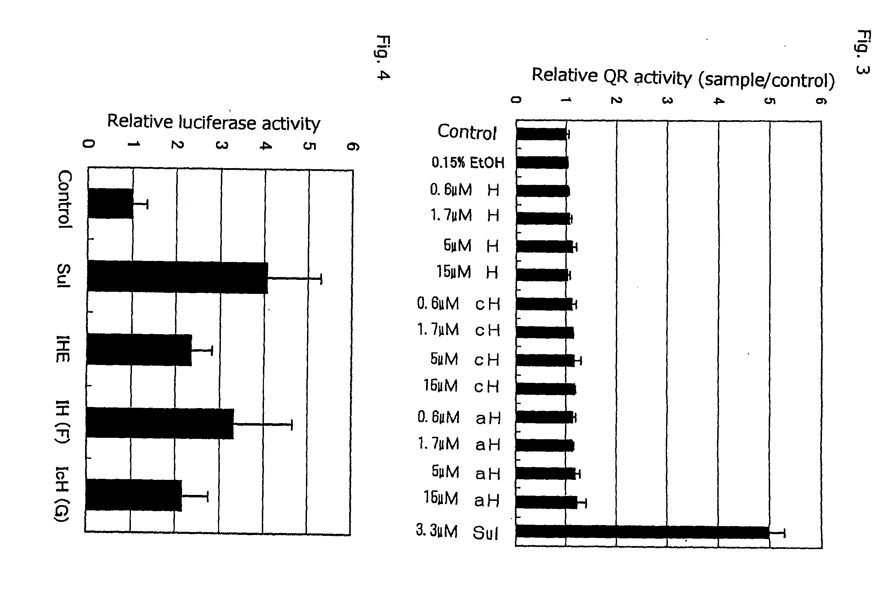

Results are shown in Figs. 1, 2, and 3.
Concentration-dependent QR inducing activity was observed with isomerized hop extract (Fig. 1) and isohumulones (i.e., isohumulone (IH), isocohumulone (IcH), and isoadhumulone (IaH)) (Fig. 2), but no clear change was observed with humulone (H), cohumulone (cH), and adhumulone (aH) (Fig. 3). Among isohumulones, isohumulone (IH) and isocohumulone (IcH) showed higher activity than isoadhumulone (IaH). Further, isohumulones can exist as the cis form or trans form. Comparison of isohumulones each having a different trans form content, i.e., 2% (IH (E)), 26% (IH (H)), and 60% (HI (F)), showed that the activity was higher in isohumulones having a higher trans form content (Fig. 2). Further, comparison of isocohumulones each having a different trans form content, i.e., 2% (IcH (D)) and 22% (IcH (G)), showed similar results.

### Example 2: Nrf2 activation in reporter assay system

To construct the reporter plasmid pGL3-mARE3A, a double-stranded oligo DNA with 3 repeats of mouse NQO1-derived ARE (antioxidant response element) to which Nrf2 is believed to bind was constructed and inserted into the MluI-NheI site of the firefly luciferase reporter vector pGL3-promoter vector (available from Promega).

The double-stranded oligo DNA containing AREs was prepared by chemically synthesizing two single-stranded oligo DNAs, 
5'-TCGACGCGTAGAGTCACAGTGAGTCGGCAAAATTAGAGTCACAGT
GAGTCGGCAAAATTAGAGTCACAGTGAGTCGGCAAAATTGCTAGCTA
G-3' (SEQ ID NO: 1) and
 and 
5'-CTAGCTAGCAATTTTGCCGACTCACTGTGACTCTAATTTTGCCGACT
CACTGTGACTCTAATTTTGCCGACTCACTGTGACTCTACGCGTCGA-3'
(SEQ ID NO: 2),
 and annealing them. Further, DNA sequence analysis confirmed that the product obtained was the plasmid of interest. Next, a mouse macrophage cell line RAW264.7 (available from American Type Culture Collection) was seeded on a 12-well plate (4 × 10⁵ cells/well), 0.8 ml of an RPMI1640 medium (Invitrogen) supplemented with 10% fetal bovine serum (HyClone) and penicillin-streptomycin (100 units/ml and 100 µg/ml, respectively; Sigma) was added, and the plate was incubated at 37°C in an atmosphere of 5% CO₂ overnight. These cells were transfected with 1.0 µg of pGL3-mARE3 and 0.01 µg of renilla luciferase reporter vector pRL-TK (Promega) for compensation using a SuperFect Transfection reagent (Qiagen). After transfection for 3 hours, the medium was freshly replaced, individual samples (i.e., 25 µg/ml isomerized hop extract, 5 µM isohumulone (IH (F)), and 5 µM isocohumulone (IcH (G))) were added, and incubation was carried out for 24 hours, after which cells were recovered. The cells were broken using a Dual-Luciferase reporter assay system (Promega) and then firefly luciferase activity and renilla luciferase activity were measured using a luminometer (Luminous CT-9000D, Dia-Iatron).

Relative luciferase activity was obtained by dividing the value for firefly luciferase activity by the value for renilla luciferase activity (n=3, mean ± standard deviation). As a positive control, 8 µM sulforaphane (Sul, available from Sigma) was used.

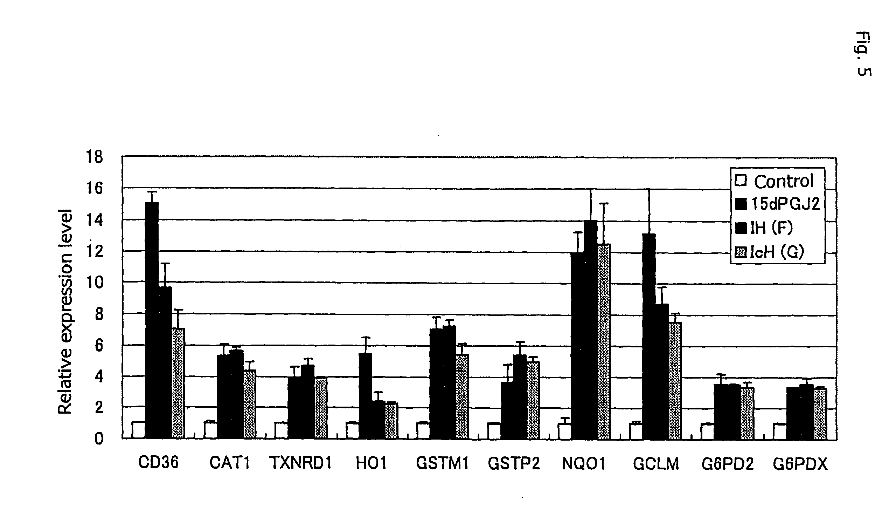

Results are shown in Fig. 4.
In this assay system, Nrf2 activation activity was examined for the samples which showed high QR inducing activity in Example 1, i.e., 25 µg/ml isomerized hop extract, 5 µM isohumulone (IH (F)), and 5 µM isocohumulone (IcH (G)). The results confirmed the activation for all samples, suggesting that such QR inducing activity occurs via Nrf2 activation.

### Example 3: Analysis of expression of various genes controlled by Nrf2

A mouse macrophage cell line RAW264.7 was seeded on a φ60 mm dish (9 × 10⁵ cells), 2 ml of a D-MEM medium (Invitrogen) supplemented with 10% fetal bovine serum (HyClone) and penicillin-streptomycin (100 units/ml and 100 µg/ml, respectively; Sigma) was added, and the dish was incubated at 37°C in an atmosphere of 5% CO₂ for 2 days. The medium was replaced with a fresh medium containing individual samples and incubation was continued for another 20 hours. Then, the total RNA was prepared using TRIzol (Invitrogen) and purified using a RNeasy Mini kit (Qiagen) and an RNase-free DNase set (Qiagen). Further, cDNA was synthesized from 5 µg of the purified total RNA using a Thermo Script RT-PCR system (Invitrogen), and the amount of mRNA expression of various genes was determined using a FastStart DNA Master SYBR Green I kit (Roche) and a Light Cycler (Roche).

The amount of expression of the various genes was corrected by the amount of expression of the housekeeping gene P0 and calculated as a relative expression level (n=2, mean ± standard deviation).

The following DNA primers used for gene expression analysis were all synthesized by Invitrogen.
Sense and antisense primers for CD36 were
5'-TGGCACAGACGCAGCCTCCTTT-3' (SEQ ID NO: 3) and
5'-TGGATTCTGGAGGGGTGATGCAAA-3 (SEQ ID NO: 4);
sense and antisense primers for catalase 1 (CAT1) were
5'-CAACAGCTTCAGCGCACCAGAGCA-3' (SEQ ID NO: 5) and
5'-TCAGGTGGCCGGCAATGTTCTCA-3' (SEQ ID NO: 6);
sense and antisense primers for thioredoxin reductase 1 (TXNRD1) were
5'-CGCGCAACTATGGCTGGAAAGTCG-3' (SEQ ID NO: 7) and
5'-TCGCCACAATCCTGTGAGGACCA-3' (SEQ ID NO: 8);
sense and antisense primers for heme oxygenase 1 (HO1) were
5'-TGCACGCCAGCCACACAGCACTAT-3' (SEQ ID NO: 9) and
5'-ACTTGGTGGGGCTGTCGATGTTCG-3' (SEQ ID NO: 10);
sense and antisense primers for glutathione S-transferase mu1 (GSTM1) were
5'-TGCCCGAAAGCACCACCTGGAT-3' (SEQ ID NO: 11) and
5'-ACCATGGCCTCTTGCCCAGGAA-3' (SEQ ID NO: 12);
sense and antisense primers for glutathione S-transferase pi2 (GSTP2) were
5'-TGCCTGGGCATCTGAAGCCTTTTG-3' (SEQ ID NO: 13) and
5'-TTGATCTTGGGCCGGGCACTGA-3' (SEQ ID NO: 14);
sense and antisense primers for NAD(P)H:quinone oxidoreductase (NQO1) were
5'-ACAGCATTGGCCACACTCCACCA-3' (SEQ ID NO: 15) and
5'-TGATGGCCCACAGAGAGGCCAAA-3' (SEQ ID NO: 16);
sense and antisense primers for glutamate cysteine ligase modifier subunit (GCLM) were
5'-TGGAATGCACCATGTCCCATGCAG-3' (SEQ ID NO: 17) and
5'-TCGGAGGCGAAGCCATGATCACA-3' (SEQ ID NO: 18);
sense and antisense primers for glucose-6-phosphate dehydrogenase 2 (G6PD2) were
5'-TGCCACCTTTGCTGCAGCTGTCCT-3' (SEQ ID NO: 19) and
5'-ACTGCCTCATTGGGCTGCATACGG-3' (SEQ ID NO: 20);
sense and antisense primers for glucose-6-phosphate dehydrogenase, X-linked (G6PDX) were
5'-TGGGTCCACCACTGCCACTTTTG-3' (SEQ ID NO: 21) and
5'-ATTGGGCTGCACACGGATGACCA-3' (SEQ ID NO: 22); and
sense and antisense primers for housekeeping gene acidic ribosomal phosphoprotein P0 were
5'-GCGTCCTGGCATTGTCTGTG-3' (SEQ ID NO: 23) and
5'-TCCTCATCTGATTCCTCCGACTC-3' (SEQ ID NO: 24).

Results are shown in Fig. 5.
As shown in Fig. 5, similar to 3 µM of 15-deoxy-D12,14-prostaglandin J₂ (15dPGJ₂) (available from Cayman Chemical) used as a positive control, 25 µM isohumulone (IH (F)) and 25 µM isocohumulone (IcH(G)) augmented the expression of the target genes whose expression is known to be increased by Nrf2, i.e., CD36, CAT1, TRNRD1, HO1, GSTM1, GSTP2, NQO1, GCLM, G6PD2, and G6PDX. From these results, it has been revealed that isohumulones activate Nrf2 and augment the expression of the genes for a series of enzymes called phase II enzymes.

### Example 4: Amount of intracellular glutathione

A mouse macrophage cell line RAW264.7 was seeded on a φ60 mm dish (7 × 10⁵ cells), 2 ml of a D-MEM medium (Invitrogen) supplemented with 10% fetal bovine serum (HyClone) and penicillin-streptomycin (100 units/ml and 100 µg/ml, respectively; Sigma) was added, and the dish was incubated at 37°C in an atmosphere of 5% CO₂ for 2 days. The medium was replaced with a fresh medium containing individual samples and incubation was continued for another 20 hours. The samples added were isohumulone (IH(F)) and isocohumulone (IcH(G)) at a concentration of 25 µM. The cells were recovered, broken by ultrasonication, and centrifuged to obtain the supernatant, and the amount of the total intracellular glutathione was measured using a glutathione assay kit (Cayman Chemical) (n=2, mean ± standard deviation). As a positive control, 15dPGJ₂ (Cayman Chemical) was used.

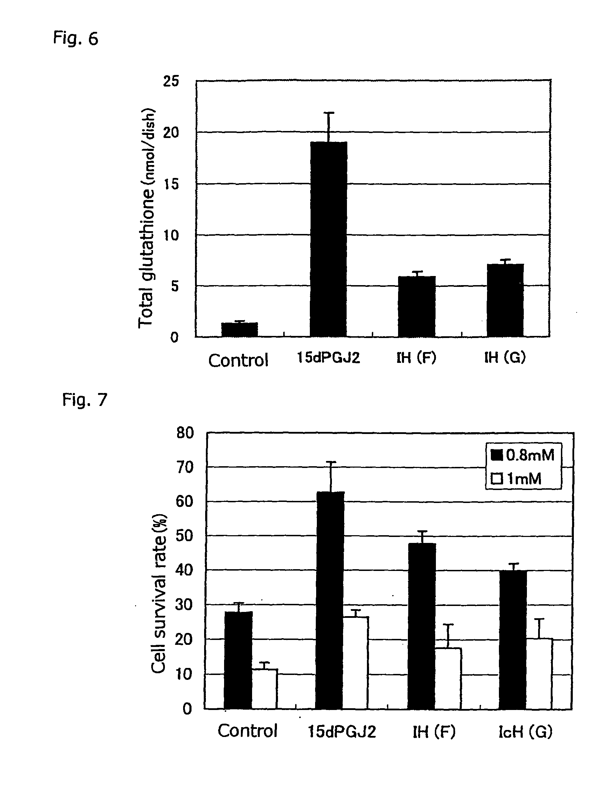

Results are shown in Fig. 6.
The effect of 25 µM isohumulone (IH(F)) and 25 µM isocohumulone (IcH(G) was examined. The results showed that they increased the amount of the total intracellular glutathione, similarly to 3 µM 15dPGJ₂. Glutathione has a function to prevent cells from reactive oxygen species or xenobiotic substances and isohumulones seem to have augmented this function.

### Example 5: Suppressive action on cell death by hydrogen peroxide

A mouse macrophage cell line RAW264.7 (6 × 10⁴ cells/well) was incubated on a 96-well plate in 100 µl of a D-MEM medium (Invitrogen) containing individual samples, 10% fetal bovine serum (HyClone), and penicillin-streptomycin (100 units/ml and 100 µg/ml, respectively; Sigma) at 37°C in an atmosphere of 5% CO₂ overnight. The samples added were isohumulone (IH(F)) and isocohumulone (IcH(G)) at a concentration of 25 µM. Hydrogen peroxide (Wako Pure Chemical Industries) was added hereto at a final concentration of 0.8 or 1.0 mM and incubation was continued for another 2 hours. Then, adsorption at 440 nm was measured using a microplate reader (Power Wave 200, Bio-Tek Instruments) with a cell growth reagent WST-1 (Roche Diagnostics). The cell survival rate for each sample was calculated by setting the value without hydrogen peroxide as 100% (n=2, mean ± standard variation). As a positive control, 15dPGJ₂ (Cayman Chemical) was used.

Results are shown in Fig. 7.
Effects of 25 µM isohumulone (IH(F)) and 25 µM isocohumulone (IcH(G)) were examined. The results confirmed that they suppressed cell death at hydrogen peroxide concentrations of both 0.8 mM and 1.0 mM, similarly to 3 µM 15dPGJ₂. From these results, isohumulones were considered to have an activity to suppress cell death caused by reactive oxygen species.

### Example 6: Suppressive action on inflammatory cytokine TNF-α production by macrophages

A mouse macrophage cell line RAW264.7 (4 × 10⁴ cells/well) was seeded on a 96-well plate, 50 ml of a D-MEM medium (Invitrogen) containing each sample, 10% fetal bovine serum (HyClone), and penicillin-streptomycin (100 units/ml and 100 µg/ml, respectively; Sigma) was added, and the plate was incubated at 37°C in an atmosphere of 5% CO₂ for 4 hours. Then 150 µl of a medium containing TPA (phorbol 12-tetradecanoate 13-acetate, Sigma), mouse IFN-γ (interferon-γ, eBioscience), and each sample was added and incubation was carried out for 24 hours. TPA and IFN-γ were added at a final concentration of 400 nM and 10 units/ml, respectively. As samples, isomerized hop extract was added at a concentration of 5, 10, 20, and 30 µg/ml and isohumulone (IH(F)) and isocohumulone (IcH(G)) were added individually at a concentration of 5, 10, 20, and 40 µM.

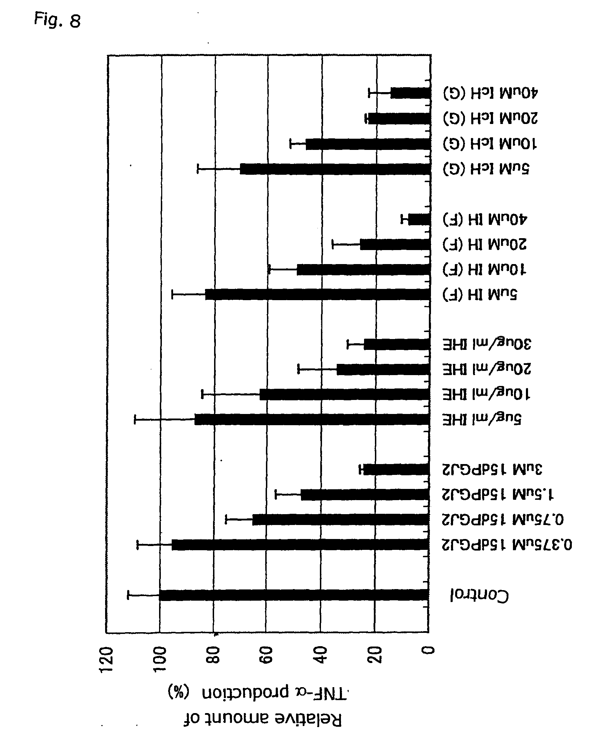

After completing incubation, the amount of TNF-α in the culture fluid was measured using a mouse TNF-α ELISA kit(eBioscience). The amount of TNF-α was expressed as a relative value by setting the value for the control as 100% (n=3, mean ± standard variation). As a positive control, 15dPGJ₂ (Cayman Chemical) was used.
Results are shown in Fig. 8.

Similar to 15dPGJ₂, isomerized hop extract, IH(F), and IcH(G) concentration-dependently suppressed the TNF-α production. From these results, isohumulones are considered to have an activity to suppress the TNF-α production by inflammation inducing substances.

### Example 7: Suppressive action on inflammation in ApoE knockout mice

ApoE knockout male mice at 8 weeks of age (available from Jackson Laboratory) were fed a high fat-high cholesterol diet (15% unsalted butter, 52.45% sucrose, 20% casein, 1% corn oil, 5% cellulose, 3.5% minerals, 1% vitamins, 0.25% choline chloride, 0.3% cystine, 1% cholesterols, and 0.5% sodium cholate) for 10 weeks and 14 weeks. At the same time, mice in a sample administration group were fed a basic diet supplemented with 1% isomerized hop extract (ISOHOPCON2, English Hop Products).

After the feeding period, blood samples were collected from the abdominal aorta under ether anesthesia and the plasma was prepared to measure the amounts of sICAM-1 and sVCAM-1 using commercially available mouse ELISA kits (sICAM-1 (Amersham Bioscience) and sVCAM-1 (R&D System)).

Results are shown in Table 1. In the table, * means p<0.05 and ** means p<0.01.
The amounts of sICAM-1 and sVCAM-1 were significantly decreased on week 10 and also tended to be decreased on week 14 by the administration of the isomerized hop extract. The sICAM-1 and sVCAM-1 are soluble forms of ICAM-1 and VCAM-1, their expression on vascular endothelial cells is augmented with inflammation, and thus they are considered to be markers suggesting inflammatory conditions. The results in Table 1 presumably suggests that isohumulones markedly suppress the expression of adhesion molecules on vascular endothelial cells in the early stage of inflammatory process and suppress adhesion and infiltration of leucocytes such as monocytes, in the progression of arteriosclerosis under high fat dieting conditions.

**Table 1**

| | Week 10 | | Week 14 | |
|---|---|---|---|---|
| | Control (n=9) | IHE (n=8) | Control (n=7) | IHE (n=9) |
| sICAM-1 (µg/ml) | 51.8 ± 8.5 | 39.6 ± 6.6* | 44.2 ± 8.9 | 40.5 ± 8.7 |
| sVCAM-1 (µg/ml) | 1.24 ± 0.19 | 0.84 ± 0.12** | 1.28 ± 0.27 | 1.13 ± 0.14 |

### Example 8: Suppressive action on the expression of adhesion molecules in vascular endothelial cells

Normal human aorta endothelial cells (HAEC, available from Cambrex) were cultured in a φ100 mm dish using 10 ml of endothelial cell medium kit-2 (Takara Shuzo) at 37°C in an atmosphere of 5% CO₂. Here, 30 µg/ml isomerized hop extract, 10 µM sulforaphane (Sul), or 10 mM N-acetyl cysteine (NAC) was allowed to act overnight. Next, after stimulating with the addition of human TNF-α (ProSpec-Tany TechnoGene) at a final concentration of 1.25 ng/ml), the cells were detached using a reagent for subculturing (Takara Shuzo) and flow cytometry analysis was carried out. Here, biotin anti-human ICAM antibody or biotin anti-human VCAM antibody (available from eBioscience) was used as a primary antibody and PE conjugated streptavidin (available from eBioscience) was used as a secondary antibody. A flow cytometer used was a FACSort analyzer (Becton Dickinson). The expression of ICAM-1 and VCAM-1 was shown as a relative value setting the value for the mean fluorescence intensity for a control to be 100%.

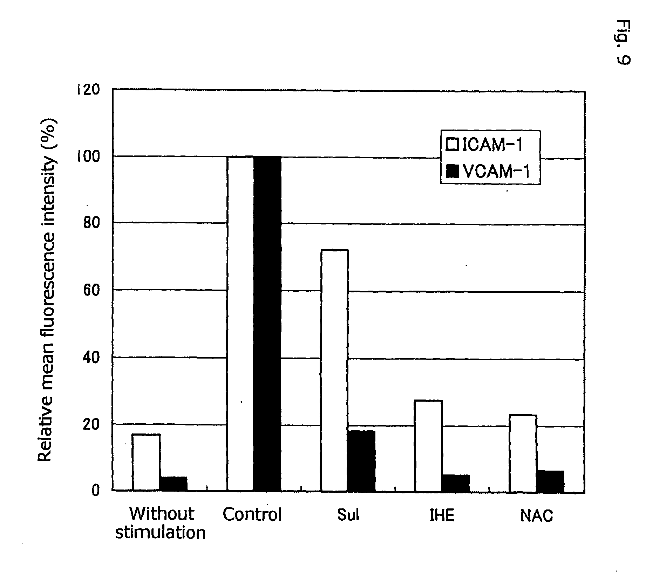

Results are shown in Fig. 9.
As shown in Fig. 9, the isomerized hop extract markedly suppressed the expression of ICAM-1 and VCAM-1 on the vascular endothelial cells by TNF-α stimulation. Such changes are also recognized with an Nrf2 activation substance, sulforaphane, and a typical antioxidant, N-acetyl cysteine (NAC). Therefore, it was suggested that isohumulones activate Nrf2 and augment cellular anti-oxidative activity, thereby being involved in the suppression of ICAM-1 and VCAM-1 expression.

### Example 9: Alteration of hepatic gene expression in mice by dietary administration

129S1/SvlmJ male mice at 5 weeks of age (obtained from Jackson Laboratory, n=5) were fed an AIN-76A diet (purchased from Dyets) supplemented with isomerized hop extract (ISOHOPCON2, English Hop Products) at stepwisely increasing concentrations from 0.1%, 0. 25% to 0.5% each for 2 days in order to accustom the mice to the taste. The animals were then fed an AIN-76A diet supplemented with 1% isomerized hop extract for one week. At the same time, mice in a control group (n=5) were fed an AIN-76A diet only.

After the feeding period, the liver was dissected under ether anesthesia, immediately frozen in liquid nitrogen and stored at -80°C until use. From the liver, total RNA was prepared using TRIzol (Invitrogen) and purified using an RNeasy Mini kit (Qiagen) and an RNase-Free DNase Set (Qiagen). Further, cDNA was synthesized from 5 mg of the purified total RNA using the Thermo Script RT-PCR system (Invitrogen) and the amount of mRNA expression of each gene was determined using FastStart DNA Master SYBR Green I (Roche) and a Light Cycler (Roche). The amount of expression of each gene was corrected by the amount of expression of the housekeeping gene P0 and calculated as a relative expression level (mean ± standard deviation (mean ± SD)).

The following DNA primers used for gene expression analysis were all synthesized by Invitrogen.
Sense and antisense primers for glutathione S-transferase mu1 (GSTM1) were
5'-TGCCCGAAAGCACCACCTGGAT-3' (SEQ ID NO: 11) and
5'-ACCATGGCCTCTTGCCCAGGAA-3' (SEQ ID NO: 12);
sense and antisense primers for NAD(P)H:quinone oxidoreductase (NQ01) were
5'-ACAGCATTGGCCACACTCCACCA-3' (SEQ ID NO: 15) and
5'-TGATGGCCCACAGAGAGGCCAAA-3' (SEQ ID NO: 16);
sense and antisense primers for glutamate cysteine ligase catalytic subunit (GCLC) were
5'-TCTGCAAAGGCGGCAACGCTGT-3' (SEQ ID NO: 25) and
5'-GCATCGGGTGTCCACATCAACTTCC-3' (SEQ ID NO: 26);
sense and antisense primers for cytochrome P450 1A1 (Cyp1A1) were
5'-TGGGGCTTGCCCTTCATTGGTC-3' (SEQ ID NO: 27)
5'-TCTGGCCGGCCCTTGAAGTCAT-3' (SEQ ID NO: 28)
sense and antisence primers for cytochrome P450 1A2 (Cyp1A2) were
5'-TCGGCCATCGACAAGACCCAGA-3' (SEQ ID NO: 29) and
5'-TGTTCACAGGTCCCGGGCTTCA-3' (SEQ ID NO: 30); and
sense and antisence primers for housekeeping gene acidic ribosomal phosphoprotein P0 were
5'-GCGTCCTGGCATTGTCTGTG-3' (SEQ ID NO: 23) and
5'-TCCTCATCTGATTCCTCCGACTC-3' (SEQ ID NO: 24).

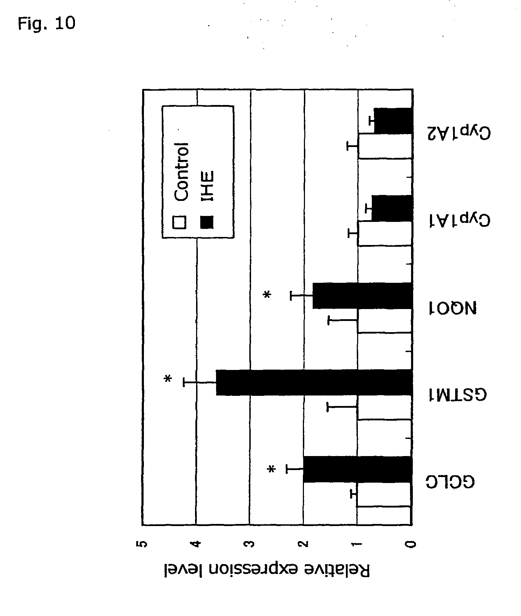

Results are shown in Fig. 10. In the figure, * means p<0.05.

In the group fed the diet containing isomerized hop extract (IHE), a significant increase in the expression of representative phase II enzymes GSTM-1, NQO1, and GCLC was recognized. From this result, it has been revealed that also when administered orally, isohumulones activate Nrf2 in the body and increase the expression of the genes for a series of enzymes called phase II enzymes. GSTM1 and NQO1 are enzymes involved in detoxification and GCLC is an enzyme directing the synthesis of an antioxidative substance, glutathione. Accordingly, it is considered that the detoxification activity and anti-oxidative activity were accelerated by the isomerized hop extract. On the other hand, the isomerized hop extract did not increase the expression of phase I enzymes represented by Cyp1A1 and Cyp1A2, which are involved in the activation of carcinogenic substances. From these results, it has also been revealed that isohumulones selectively increase the expression of phase II enzymes in the metabolism of the detoxification system.

## Claims

1. A composition for use in the treatment, prevention or amelioration of a disease or condition which is treatable, preventable, or ameliorable by activation of transcription factor Nrf2 (NF-E2 related factor 2), comprising isohumulones or isomerized hop extract as an active ingredient.

2. The composition according to claim 1, wherein the disease or condition which is treatable, preventable, or ameliorable by the activation of transcription factor Nrf2 is oxidative stress, detoxification of xenobiotic substances, chronic inflammation, or a disease or condition related thereto.

3. The composition according to claim 2, wherein the disease or condition is cerebral nerve degenerative disease, eye disease, skin disease, asthma, or a disease or condition related thereto.

4. The composition according to claim 1 or 2, for use in the prevention or suppression of metastasis or infiltration of cancer cells.

5. The composition according to claim 1 or 2, for use in the treatment, prevention, or amelioration of inflammation of the arterial wall which leads to arteriosclerosis.

6. The composition according to any one of claims 1 to 5, wherein the isohumulones are selected from the group consisting of isohumulone, isoadhumulone, isocohumulone, and a combination thereof.

7. The composition according to any one of claims 1 to 6, wherein the isohumulones are trans-isohumulones.

8. The composition according to any one of claims 1 to 5, wherein the active ingredient is isomerized hop extract.

9. The composition according to any one of claims 1 to 8, which is provided in the form of food.

10. The composition according to claim 9, wherein the food is in the form of drink.

11. The composition according to claim 10, wherein the drink is a non-alcoholic drink.

12. The composition according to any one of claims 9 to 11, wherein the food is a health food, functional food, food for specified health use, nutrition supplement food, food claiming to reduce disease risk, and food for patients.

13. The composition according to any one of claims 1 to 8, which is provided in the form of medicine.

14. The composition according to claim 13, which is provided as an oral agent or an agent for external use.

15. A transcription factor Nrf2 activating agent, comprising isohumulones or isomerized hop extract as an active ingredient.

16. The activating agent according to claim 15, wherein the isohumulones are selected from the group consisting of isohumulone, isoadhumulone, isocohumulone, and a combination thereof.

17. The activating agent according to claim 15 or 16, wherein the isohumulones are trans-isohumulones.

18. The activating agent according to any one of claims 15 to 17, which is provided in the form of food.

19. The activating agent according to any one of claims 15 to 18, for use as an antioxidant, detoxification agent, or anti-inflammatory agent.

20. The activating agent according to any one of claims 15 to 19, for use in increasing the amount of intracellular glutathione.

21. The activating agent according to any one of claims 15 to 19, for use in suppressing cell death by reactive oxygen species.

22. A food for use in the treatment, prevention, or amelioration of a disease or condition which is treatable, preventable, or ameliorable by the activation of transcription factor Nrf2, comprising an effective amount of isohumulones or isomerized hop extract.

23. A food for antioxidation, detoxification, or anti-inflammation, comprising an effective amount of isohumulones or isomerized hop extract.

24. A food for use in the activation of transcription factor Nrf2, comprising an effective amount of isohumulones or isomerized hop extract.

25. A food which carries an indication for its function to treat, prevent, or ameliorate a disease or condition which is treatable, preventable, or ameliorable by the activation of transcription factor Nrf2.

26. A food which carries an indication for its function in antioxidation, detoxification, or anti-inflammation, comprising an effective amount of isohumulones or isomerized hop extract.

27. A food which carries an indication for its function to activate the transcription factor Nrf2, comprising an effective amount of isohumulones or isomerized hop extract.

28. The food according to any one of claims 25 to 27, wherein the indication for its function is placed onto the product itself, a container, a package, an instruction, a package insert, or an advertisement.

29. The food according to claim 22 or 25, wherein the disease or condition which is treatable, preventable, or ameliorable by the activation of transcription factor Nrf2 is oxidative stress, detoxification of xenobiotic substances, chronic inflammation, or a disease or condition related thereto.

30. The food according to claim 29, wherein the disease or condition is cerebral nerve degenerative disease, skin disease, eye disease, asthma, or a disease or condition related thereto.

31. The food according to claim 22 or 25, for use in the prevention or suppression of metastasis or infiltration of cancer cells.

32. The food according to claim 22 or 25, for use in the treatment, prevention, or amelioration of inflammation of the arterial wall which leads to arteriosclerosis.

33. The food according to any one of claims 22 to 32, wherein the isohumulones are selected from the group consisting of isohumulone, isoadhumulone, isocohumulone, and a combination thereof.

34. The food according to any one of claims 22 to 33, wherein the isohumulones are trans-isohumulones.

35. The food according to any one of claims 22 to 34, wherein the isohumulones or isomerized hop extract are provided in the range of 3 mg to 3000 mg per day per adult as the amount of isohumulones.

36. The food according to any one of claims 22 to 35, which is in the form of drink.

37. The food according to claim 36, wherein the drink is a non-alcoholic drink.

38. The food according to claim 37, wherein the drink is a tea drink.

39. The food according to any one of claims 22 to 38, which is a health food, functional food, food for specified health use, nutrition supplement food, food claiming to reduce disease risk, and food for patients.

40. A method of treating, preventing, or ameliorating a disease or condition which is treatable, preventable, or ameliorable by the activation of transcription factor Nrf2, comprising the step of administering or feeding a therapeutically effective amount of isohumulones or isomerized hop extract to a mammal.

41. The method according to claim 40, wherein the disease or condition which is treatable, preventable, or ameliorable by the activation of transcription factor Nrf2 is oxidative stress, detoxification of xenobiotic substances, chronic inflammation, or a disease or condition related thereto.

42. The method according to claim 41, wherein the disease or condition is cerebral nerve degenerative disease, eye disease, skin disease, asthma, or a disease or condition related thereto.

43. The method according to claim 40 or 41, for use in the prevention or suppression of metastasis or infiltration of cancer cells.

44. The method according to claim 40 or 41, for use in the treatment, prevention, or amelioration of inflammation of the arterial wall which leads to arteriosclerosis.

45. The method according to any one of claims 40 to 44, wherein the isohumulones are selected from the group consisting of isohumulone, isoadhumulone, isocohumulone, and a combination thereof.

46. The method according to any one of claims 40 to 45, wherein the isohumulones are trans-isohumulones.

47. The method according to any one of claims 40 to 44, wherein the active ingredient is isomerized hop extract.

48. The method according to any one of claims 40 to 47, comprising the step of feeding the isohumulones or isomerized hop extract in the form of food.

49. A method of activating the transcription factor Nrf2, comprising the step of administering an effective amount of isohumulones or isomerized hop extract to a mammal.

50. Use of isohumulones or isomerized hop extract for manufacturing a composition for use in treating, preventing, or ameliorating a disease of condition which is treatable, preventable, or ameliorable by the activation of transcription factor Nrf2.

51. Use of isohumulones or isomerized hop extract for manufacturing an Nrf2 activating agent.

52. Use of isohumulones or isomerized hop extract for manufacturing an antioxidative agent, detoxification agent, or anti-inflammatory agent.


SEQUENCE LISTING

<110>  Kirin Beer Kabushiki Kaisha

<120>  Compsitions and foods for activating Nrf2

<130>  156848q

<160>  30

<170>  PatentIn version 3.1

<210>  1
<211>  93
<212>  DNA
<213>  Artificial

<220>
<223>  origo DNA containg antioxidant response element

<400>  1
tcgacgcgta gagtcacagt gagtcggcaa aattagagtc acagtgagtc ggcaaaatta      60

gagtcacagt gagtcggcaa aattgctagc tag                                   93


<210>  2
<211>  93
<212>  DNA
<213>  Artificial

<220>
<223>  origo DNA containg antioxidant response element

<400>  2
ctagctagca attttgccga ctcactgtga ctctaatttt gccgactcac tgtgactcta      60

attttgccga ctcactgtga ctctacgcgt cga                                   93


<210>  3
<211>  22
<212>  DNA
<213>  Artificial

<220>
<223>  PCR primer

<400>  3
tggcacagac gcagcctcct tt                                                22

<210>  4
<211>  24
<212>  DNA
<213>  Artificial

<220>
<223>  PCR primer

<400>  4
tggattctgg aggggtgatg caaa                                              24

<210>  5
<211>  24
<212>  DNA
<213>  Artificial

<220>
<223>  PCR primer

<400>  5
caacagcttc agcgcaccag agca                                              24

<210>  6
<211>  23
<212>  DNA
<213>  Artificial

<220>
<223>  PCR primer

<400>  6
tcaggtggcc ggcaatgttc tca                                               23

<210>  7
<211>  24
<212>  DNA
<213>  Artificial

<220>
<223>  PCR primer

<400>  7
cgcgcaacta tggctggaaa gtcg                                              24


<210>  8
<211>  23
<212>  DNA
<213>  Artificial

<220>
<223>  PCR primer

<400>  8
tcgccacaat cctgtgagga cca                                               23


<210>  9
<211>  24
<212>  DNA
<213>  Artificial

<220>
<223>  PCR primer

<400>  9
tgcacgccag ccacacagca ctat                                              24


<210>  10
<211>  24
<212>  DNA
<213>  Artificial

<220>
<223>  PCR primer

<400>  10
acttggtggg gctgtcgatg ttcg                                              24


<210>  11
<211>  22
<212>  DNA
<213>  Artificial

<220>
<223>  PCR primer

<400>  11
tgcccgaaag caccacctgg at                                                22

<210>  12
<211>  22
<212>  DNA
<213>  Artificial

<220>
<223>  PCR primer

<400>  12
accatggcct cttgcccagg aa                                                22

<210>  13
<211>  24
<212>  DNA
<213>  Artificial

<220>
<223>  PCR primer

<400>  13
tgcctgggca tctgaagcct tttg                                              24

<210>  14
<211>  22
<212>  DNA
<213>  Artificial

<220>
<223>  PCR primer

<400>  14
ttgatcttgg gccgggcact ga                                                22

<210>  15
<211>  23
<212>  DNA
<213>  Artificial

<220>
<223>  PCR primer

<400> 15
acagcattgg ccacactcca cca 23

<210> 16
<211> 23
<212> DNA
<213> Artificial

<220>
<223> PCR primer

<400> 16
tgatggccca cagagaggcc aaa 23

<210> 17
<211> 24
<212> DNA
<213> Artificial

<220>
<223> PCR primer

<400> 17
tggaatgcac catgtcccat gcag 24

<210> 18
<211> 23
<212> DNA
<213> Artificial

<220>
<223> PCR primer

<400> 18
tcggaggcga agccatgatc aca 23

<210> 19
<211> 24
<212> DNA
<213> Artificial

<220>
<223> PCR primer

<400>  19
tgccaccttt gctgcagctg tcct                                              24


<210>  20
<211>  24
<212>  DNA
<213>  Artificial

<220>
<223>  PCR primer

<400>  20
actgcctcat tgggctgcat acgg                                              24


<210>  21
<211>  23
<212>  DNA
<213>  Artificial

<220>
<223>  PCR primer

<400>  21
tgggtccacc actgccactt ttg                                               23


<210>  22
<211>  23
<212>  DNA
<213>  Artificial

<220>
<223>  PCR primer

<400>  22
attgggctgc acacggatga cca                                               23


<210>  23
<211>  20
<212>  DNA
<213>  Artificial

<220>
<223>  PCR primer

<400>  23
gcgtcctggc attgtctgtg                                                    20

<210>  24
<211>  23
<212>  DNA
<213>  Artificial

<220>
<223>  PCR primer

<400>  24
tcctcatctg attcctccga ctc                                                23

<210>  25
<211>  22
<212>  DNA
<213>  Artificial

<220>
<223>  primer

<400>  25
tctgcaaagg cggcaacgct gt                                                 22

<210>  26
<211>  25
<212>  DNA
<213>  Artificial

<220>
<223>  primer

<400>  26
gcatcgggtg tccacatcaa cttcc                                              25

<210>  27
<211>  22
<212>  DNA
<213>  Artificial

<220>
<223>  primer

<400>  27
tggggcttgc ccttcattgg tc                                                22

<210>  28
<211>  22
<212>  DNA
<213>  Artificial

<220>
<223>  primer

<400>  28
tctggccggc ccttgaagtc at                                                22

<210>  29
<211>  22
<212>  DNA
<213>  Artificial

<220>
<223>  primer

<400>  29
tcggccatcg acaagaccca ga                                                22

<210>  30
<211>  22
<212>  DNA
<213>  Artificial

<220>
<223>  primer

<400>  30
tgttcacagg tcccgggctt ca                                                22